# EUROPEAN PATENT SPECIFICATION

(11) **EP 3 193 848 B1**
(45) Date of publication and mention of the grant of the patent: **15.04.2020**
(21) Application number: 15842389.7
(22) Date of filing: 16.09.2015
(51) Int. Cl.: A61K 9/68, A23L 27/00, A23L 27/20

(54) **PRODUCTS CONTAINING NOVEL FLAVOR COMPOSITION**
PRODUKTE MIT NEUARTIGER GESCHMACKSZUSAMMENSETZUNG
PRODUITS CONTENANT DE NOUVELLES COMPOSITIONS AROMATISANTES

(30) Priority: 16.09.2014 US 201462051072 P
(43) Date of publication of application: 26.07.2017
(73) Proprietor: Wm. Wrigley Jr. Company, Chicago, IL 60642 (US)
(72) Inventor: JOHNSON, Sonya S., Rushville, IL 62681 (US); SHELDON, Gloria T., Valparaiso, IN 46385 (US); STICKA, Shanna, Chicago, IL 60613 (US); TRAN, Lisa, Chicago, IL 60656 (US); CHIDICHIMO, Darci, Corwn Point, IN 46307 (US); OH, Donna, Fircrest, WA 98466 (US)
(74) Representative: Haseltine Lake Kempner LLP
(86) International application number: PCT/US2015/050505
(87) International publication number: WO 2016/044470

(56) References cited:
- WO-A1-2012/016161
- WO-A1-2012/024410
- WO-A2-2005/048965
- US-A- 4 724 151
- US-A1- 2004 071 757
- US-A1- 2006 280 852
- US-A1- 2007 003 638
- US-A1- 2007 256 265
- US-A1- 2012 135 094
- US-A1- 2014 004 223
- LONG. ET AL.: 'MACH MINT, YAKOV SMIRNOFF', [Online] 21 March 2010, page 1, XP055419423 Retrieved from the Internet: <URL:HTTP://JIMLONGSGARDEN.BLOGSPOT.NL/2010 /03/THERES-NEW-MINT-ON-WORLD-MARKET-THIS.HT ML>

## Description

### CROSS-REFERENCE TO RELATED APPLICATIONS

This application claims priority to U.S. Provisional Patent Application Serial No. 62/051,072, filed September 16, 2014.

### FIELD

The presently disclosed subject matter generally relates to consumer products containing a novel flavor composition including one or more mint cultivar oils.

### BACKGROUND

Mint plants are cultivated as industrial crops in order to recover essential oils. These oils can be incorporated into a variety of consumer products. For example, mint oil is often used in oral (orally introduced) products, such as chewing gum, confections, lozenges, edible film, pastilles, dentifrice, toothpaste, mouthwash, mouth spray, tobacco, and pharmaceuticals. In particular, peppermint oil and spearmint oil have dominated the commercial market in these consumer products.

Due to hybridization, there are several thousand mint cultivars, which have not been explored or analyzed for commercial use. These new non-commercial mint cultivars can provide new essential oils that hold the potential to provide different organoleptic qualities. Since the commercial market could benefit from new flavor profiles in consumer products, e.g., chewing gum, it is desirable to provide novel flavor compositions. For instance, there remains a need for new mint flavor profiles in oral products with improved flavor and breath freshening qualities. The presently disclosed subject matter addresses this need as discussed in detail below.

WO 2012/024410 provides for a chewing gum product that imparts mouth-moistening perception to an individual upon consumption.

WO 2012/016161 provides for a delivery system that provides improved control and/or delay of release or one or more active agents.

US 2006/0280852 provides for a composition which includes: at least one characterizing vanilla flavor and at least one contributory mint flavor.

WO 2005/048965 relates to the composition of, and methods of using an oral product, specifically intended for breath freshening properties, formulated to freshen breath of consumers of tobacco products.

US 4,724, 151 provides a chewing gum composition which alleviates the adhesion properties of conventional formulations and aids in initial and long lasting flavor impact and perceived breath freshening power without higher level of flavor oils.

### SUMMARY

The presently disclosed subject matter is directed to a composition comprising one or more mint cultivar oils selected from the group consisting of ginger mint, blue balsam tea mint (blue balsam mint), macho mint, julep mint, banana mint, sweet pear mint, lavender mint, and combinations thereof, wherein the one or more mint cultivar oils is present in an amount of about 0.01 % to about 5.0 % by weight of a product composition, wherein the one or more mint cultivar oils is in an amount effective to provide a primary sensory note selected from the group consisting of spearmint, peppermint, or combinations thereof, and wherein the composition further comprises peppermint and/or spearmint.

In certain embodiments, the mint cultivar oil is ginger mint. In certain embodiments, the mint cultivar oil is blue balsam tea mint. In certain embodiments, the mint cultivar oil is macho mint. In certain embodiments, the mint cultivar oil is julep mint. In certain embodiments, the mint cultivar oil is banana mint. In certain embodiments, the mint cultivar oil is sweet pear mint. In certain embodiments, the mint cultivar oil is lavender mint.

The composition further comprises peppermint and/or spearmint. In certain embodiments, the mint cultivar oils comprise macho mint, julep mint, and combinations thereof, wherein the composition further comprises peppermint. In certain embodiments, the mint cultivar oils comprise ginger mint, lavender mint, and combinations thereof, wherein the composition further comprises peppermint.

In certain embodiments, the composition further comprises a cooling agent. In certain embodiments, the cooling agent is selected from the group consisting of menthol, N-ethyl-p-menthane-3-carboxamide, N,2,3-trimethyl-2-isopropyl-butanamide, ethyl 3-(p-menthane-3-carboxamido) acetate, [(2-isopropyl-5-methyl-cyclohexanecarbonyl)- amino]-acetic acid isopropyl ester, menthyl glutarate, menthyl succinate, menthyl lactate, 3-1-menthoxypropane-1,2-diol, eucaplyptol, and isopulegol, and combinations thereof.

In certain embodiments, the composition comprises between 0.01% to 5.0% cooling agent by weight of the composition. In certain embodiments, the composition comprises between 0.02% to 2.0% cooling agent by weight of the composition.

In certain embodiments, the composition is formulated into a consumer product selected from the group consisting of chewing gum, confection, lozenge, pastilles, dentifrice, toothpaste, mouthwash, mouth spray, tobacco, pharmaceuticals, and edible films.

In certain embodiments, the consumer product is chewing gum. In a specific embodiment, the one or more mint cultivar oils is encapsulated.

In certain embodiments, the composition further comprises a fruit flavor. In certain embodiments, the composition further comprises a sweetener.

In certain embodiments, the one or more mint cultivar oils is present in an amount effective to provide a secondary sensory note selected from the group consisting of vanilla, peppery, chalky, green, leafy, piney, fish oil, citrus, lemon, creamy, woody, herbal, lemon pith, plasticy, metallic, buttermint, spicy, honey, green tea, lemon zest and combinations thereof.

Other embodiments are disclosed, and features of each of the embodiments can be used alone or together in combination. Additional features and advantages of the disclosed embodiments are described in, and will be apparent from, the following Detailed Description.

### DETAILED DESCRIPTION

The present invention will now be further described. In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

### 1. Definitions

The terms used in this specification generally have their ordinary meanings in the art, within the context of this disclosed subject matter and in the specific context where each term is used. Certain terms are discussed below, or elsewhere in the specification, to provide additional guidance to the practitioner in describing the compositions and methods of the disclosed subject matter and how to make and use them.

Unless otherwise specified, all percentages herein are weight percentages. Although some terms are referred to in the singular, it is understood that such references may also encompass the plural. Finally, all references cited herein are incorporated by reference.

As used herein, the use of the word "a" or "an" when used in conjunction with the term "comprising" in the claims and/or the specification may mean "one," but it is also consistent with the meaning of "one or more," "at least one," and "one or more than one." Still further, the terms "having," "including," "containing" and "comprising" are interchangeable and one of skill in the art is cognizant that these terms are open ended terms.

The term "about" or "approximately" means within an acceptable error range for the particular value as determined by one of ordinary skill in the art, which will depend in part on how the value is measured or determined, i.e., the limitations of the measurement system. For example, "about" can mean within 3 or more than 3 standard deviations, per the practice in the art. Alternatively, "about" can mean a range of up to 20%, preferably up to 10%, more preferably up to 5%, and more preferably still up to 1% of a given value. All percentages and ratios used herein are by weight of the total composition and all measurements made are at 25°C, unless otherwise designated.

As used herein, "flavor" shall include aroma, odor and/or taste. The flavor composition can be in a variety of forms including but not limited to a liquid, dry powder, spray, paste, suspension, other solid form, and any combination thereof. The flavor can be a natural composition, an artificial composition or any combination thereof.

As used herein, "food product" or "food product composition" includes ingestible products including but not limited to human foods, animal or pet foods, pharmaceutical products, and consumer products.

As used herein "admixing the product or food product with a flavor composition" refers to the process where the flavor is mixed with or added to the completed product or mixed with some or all of the components of the product during product formation or some combination of these steps. When used in the context of admixing the term "product" refers to the product or any of its components. This admixing step can include a process selected from the step of adding the flavor to the product, spraying the flavor on the product, coating the flavor on the product, suspending the product in the flavor, painting the flavor on the product, pasting the flavor on the product, encapsulating the product with the flavor, mixing the flavor with the product and any combination thereof. The flavor composition can be a liquid, dry powder, spray, paste, suspension and any combination thereof.

In the context of the presently disclosed subject matter, chewing gum refers to chewing gum, bubble gum and the like. As used herein, the term "chewing gum" refers to a flavored substance intended for chewing. The term as used herein also includes bubble gum and confectionery products containing chewing gum. In certain embodiments, chewing gum forms include, but are not limited to, tablets, sticks, solid balls, hollow balls, cut and wrap, and pellets or pillows. Unless otherwise specified, all percentages used herein are weight percent. As used herein, chewing gum contains a water insoluble base portion and a water-soluble bulk portion.

As used herein, "physiological cooling agents" encompasses any number of physiological cooling agents but does not include traditional flavor-derivatives such as menthol or menthone. Preferred physiological agents provide a cooling effect without imparting perceptible flavor of their own. Several known compounds have what can be characterized as a "cooling" activity, and are referred to in the art as "physiological cooling agents." Physiological cooling agents are perceived as cold or cool when contacted with the human body and, in particular, with the mucous membranes of the mouth, nose and throat.

### 2. Mint Cultivars Oils

The presently disclosed subject matter provides unique mint oils that are obtained from a variety of plants. Each of the cultivated mint varietals has a different flavor profile of interest. For example, each of ginger mint, blue balsam tea mint, macho mint, julep mint, banana mint, sweet pear mint, lavender mint, spearmint, and peppermint cultivars produce essential oils with unique flavor profiles as discussed below.

Ginger mint (*mentha x gracilis*) oil comes from the ginger mint plant. Synonyms for this plant include red stemmed mint, doublemint, Vietnamese mint, Slender, Scotch, Red, Austrian, and Golden apple. Ginger mint is a perennial plant that grows about 15-inches in height. Its heart-shaped leaves are light green and variegated with gold. It has strong fruity flavored leaves and produces inter-nodal lavender flowers. Ginger mint oil's FEMA GRAS number is 4811.

Table 1 shows a comparison between spearmint, peppermint, and ginger mint plant morphology and yield. As noted in Table 1, Ginger mint's plant morphology differs from the morphology of spearmint and peppermint plants in numerous aspects including plant height, leaf color, flower location, and leaf shape.

**Table 1. Plant morphology and yield**

| | Native Spearmint (*Mentha spicata*) (aka Spearmint) | Black Mitcham (*Mentha piperita*) (aka Peppermint) | Ginger Mint (*Mentha x gracilis*) |
|---|---|---|---|
| Plant Height average (inches) | 30 | 24 | 15 |
| Leaf Shape | Ovate | Oblong | Cordate |
| Leaf Color | Light Green-Green | Dark Green-Green | Green w/ yellow variegation |
| Stem Color | Green | Reddish-Green | Red |
| Flower Location | Axil | Axil | Inter-nodal |
| Flower Color | White | Dark Purple | Lavender |
| Greenhouse Yield | 0.40% | 0.30% | 0.35% |

The ginger mint oil is distinct from spearmint and peppermint oil in its sensory-organoleptic properties as shown by Table 2. Specifically, ginger mint has spicy, floral, and crisp notes.

**Table 2. Sensory-organoleptic analysis of essential oils by trained flavorists**

| | Native Spearmint (*Mentha spicata*) | Black Mitcham (*Mentha piperita)* | Ginger Mint (*Mentha x gracilis*) |
|---|---|---|---|
| Sensory-organoleptic analysis | Fresh, carvone | Sweet, mentholic | Spicy, floral, crisp |

Table 3 provides the specific composition of each of peppermint, ginger mint, and spearmint oil. Table 4 provides the chiral constituents of spearmint and ginger mint oils. As apparent in the Tables below, ginger mint oil's composition is significantly different than spearmint and peppermint. It is notable that ginger mint oil has a high linalool content, which can range from 40% to 65% of the oil, has no menthol, and has very little carvone; whereas peppermint tends to have high menthol, and spearmint tends to have high carvone. Ginger mint oil does not have the significant cooling components (like menthol, menthone, isomenthone, isopulegol, etc.) that peppermint oil has and therefore imparts a very different flavor profile as noted by trained flavorists in Table 2.

**Table 3. Composition of Peppermint, Ginger Mint and Spearmint Oils**

| | Black Mitcham (*Mentha piperita)* | Ginger Mint (*Mentha x gracilis*) | | Native Spearmint (*Mentha spicata*) |
|---|---|---|---|---|
| Isovaleraldehyde | 0.01 | 0.01 | Isovaleraldehyde | 0.15 |
| α-Pinene | 0.87 | 2.41 | α-Pinene | 0.76 |
| β-Pinene | 1.23 | 8.70 | 2,5-Diethyl THF | 0.08 |
| Sabinene | 0.59 | 1.39 | Camphene | 0.02 |
| Myrcene | 0.29 | 1.49 | β-Pinene | 0.65 |
| α-Terpinene | 0.10 | 0.65 | Sabinene | 0.43 |
| 1-Limonene | 2.05 | 1.64 | Myrcene | 2.61 |
| 1, 8-Cineole | 3.58 | 2.40 | α-Phellandrene | 0.04 |
| trans-Ocimene | - | 8.22 | α-Terpinene | 0.30 |
| cis-Ocimene | 0.46 | - | 1-Limonene | 11.74 |
| γ-Terpinene | 0.29 | 7.15 | 1, 8-Cineole | 1.71 |
| p-Cymene | 0.07 | 2.56 | cis-Ocimene | 0.21 |
| Terpinolene | 0.10 | 0.19 | γ-Terpinene | 0.50 |
| 3-octanol | 0.22 | 0.36 | p-Cymene | 0.05 |
| 1-Octen-3-ol | 0.04 | 0.02 | Terpinolene | 0.17 |
| trans-sabinene hydrate | 1.23 | 0.07 | 3-Octyl acetate | - |
| 1-Menthone | 14.09 | 0.16 | cis-3-Hexenol | 0.02 |
| Menthofuran | 7.09 | 0.32 | 3-Octanol | 0.87 |
| Isomenthone | 1.84 | - | trans-2-Hexenol | - |
| β-bourbonene | 0.29 | 0.07 | trans-sabinene hydrate | 0.88 |
| Linalool | 0.37 | 41.81 | 1-Menthone | 0.12 |
| cis-sabinene hydrate | 0.02 | - | 2-Ethyl-1-hexanol | 0.01 |
| Menthyl acetate | 9.16 | - | cis-3-Hexyl isovalerate | 0.03 |
| Isopulegol | 0.05 | - | Isomenthone | 0.10 |
| Isopulegone | 0.50 | - | β-Bourbonene | 1.43 |
| Neomenthol | 3.33 | - | Linalool | - |
| Terpinen-4-ol | 0.22 | 0.11 | Menthyl acetate | 0.07 |
| β-caryophyllene | 1.84 | 5.77 | Isopulegol | 0.19 |
| N eoisomenthol | 0.87 | - | Neomenthol | 0.11 |
| 1-Menthol | 41.07 | - | Terpinen-4-ol | 0.93 |
| Pulegone | 0.11 | - | β-Caryophyllene | 0.83 |
| Isomenthol | 0.24 | - | cis-Dihydrocarvone | 1.01 |
| β-Farnesene | 0.54 | 0.16 | trans-Dihydro carvone | - |
| α-Humulene | 0.11 | 0.23 | 1-Menthol | 0.35 |
| α-Terpineol | 0.16 | 0.64 | Pulegone | 0.72 |
| Germacrene-D | 2.43 | 3.00 | β-Farnesene | 0.36 |
| Piperitone | 0.31 | 0.57 | Dihydrocarvyl Acetate | 0.05 |
| 1-Carvone | 0.50 | 0.47 | β-Bisabolene | - |
| Piperitone oxide | - | 0.57 | β-Cubebene | - |
| Viridiflorol | 0.45 | - | α-Terpineol | 0.05 |
| Thymol | - | 2.53 | Germacrene-D | 0.50 |
| Eugenol | - | 0.15 | Piperitone | 0.16 |
| Isoamyl phenylacetate | - | 0.67 | 1-Carvone | 66.49 |
| Carvacrol | - | 0.17 | Dihydrocarveol | 0.05 |
| | | | Carvyl Acetate | 0.29 |
| | | | Carveol I | 0.40 |
| | | | Carvone Oxide | 0.09 |
| | | | Carveol II | 0.26 |
| | | | cis-Jasmone | 0.28 |
| | | | Viridiflorol | - |

**Table 4. Chiral constituents of Spearmint and Ginger Mint Oil**

| Chiral Compounds | Native Spearmint (*Mentha spicata*) | Ginger Mint (*Mentha x gracilis*) |
|---|---|---|
| (1S)-(-)-a-PINENE | 0.60 | 0.92 |
| (1R)-(+)-a-PINENE | 0.28 | 0.48 |
| (+)-SABINENE | 0.29 | 0.51 |
| (-)-SABINENE | 0.19 | 0.68 |
| (+)-B-PINENE | 0.54 | 4.44 |
| (-)-B-PINENE | 0.42 | 2.11 |
| (S)-(-)-LIMONENE | 12.55 | 4.43 |
| (R)-(+)-LIMONENE | - | - |
| (1R, 4S)-(-)-MENTHONE | 0.13 | 0.71 |
| (1R, 4R)-(+)-ISOMENTHONE | 0.02 | 0.10 |
| (3R)-(-)-LINALOOL | - | 31.84 |
| (3R)-(-)-LINALYL ACETATE | - | 1.90 |

Blue balsam tea mint (*Mentha x piperata cv*.) oil (or blue balsam mint) comes from the blue balsam tea mint plant and is considered a peppermint-like cultivar. The blue balsam tea mint oil has a flavor profile of peppermint, herbal and honey notes.

Macho Mint (*Mentha spicata*) oil comes from the macho mint plant and is a spearmint cultivar. Macho mint oil has a flavor profile of fresh, spearmint, and leaf-like notes.

Julep Mint (*Mentha cv*.) oil comes from the julep mint plant and is a spearmint cultivar. Julep mint oil has a flavor profile of full, green, leaf-like notes.

Lavender Mint (*Mentha citrata cv*.) oil comes from the lavender mint. Lavender mint oil has lavender, floral flavor notes.

Banana Mint (*Mentha arvensis cv*.) oil comes from the banana mint plant. Banana mint oil gives buttery, banana, vanilla, and mint flavor notes.

Sweet Pear Mint (*Mentha cv*.) oil comes from the sweet pear mint plant. The sweet pear mint oil gives fruity, herbal and mint flavor notes.

Table 5 shows a comparison of plant morphology and yield between spearmint, peppermint, macho mint, julep mint, blue balsam tea mint, and ginger mint plants.

**Table 5. Plant morphology and yield of Mentha cultivars.**

| | Native Spearmint (*M*. *spicata*) | Macho Mint (*M. spicata*) | Julep Mint (*Mentha cv*) | Black Mitcham Peppermint (*M*. *piperita*) | Ginger Mint (*M*. *x gracilis)* | Blue Balsam tea mint (*M*. *x piperita cv)* |
|---|---|---|---|---|---|---|
| Plant Height (inches) | 30 | 36 | 36 | 24 | 15 | 24 |
| Leaf Shape | Ovate | Ovate | Ovate | Oblong | Cordate | Ovate |
| Leaf Color | Light Green-Green | Light Green-Green | Light Green-Green | Dark Green-Green | Green w/ yellow variegation | Dark Green-Green |
| Stem Color | Green | Green | Green | Reddish-Green | Red | Red |
| Flower Location | Axil | Axil | Axil | Axil | Inter-nodal | Axil |
| Flower Color | White | Pinkish-white | White | Dark Purple | Lavender | Pinkish-Purple |
| Greenhouse Yield | 0.40% | 0.66% | 0.58% | 0.30% | 0.35% | 0.42% |

Table 6 shows the composition analysis of macho mint, julep mint, ginger mint, and blue balsam tea mint oils in comparison to the spearmint and peppermint oils. The units are percent of total Gas Chromatography - Flame Ionization Detector. As shown in Table 6, a number of differences exist between the different oils.

**Table 6. Composition analysis of new Mentha cultivars**

| | Native Spearmint (*M*. *spicata)* | Macho Mint *(M. spicata*) | Julep Mint (*Mentha cv*) | Black Mitcham Peppermint (*M*. *piperita*) | Ginger Mint (*M*. *x gracilis)* | Blue Balsam tea mint (*M*. *x piperita cv)* |
|---|---|---|---|---|---|---|
| Isovaleraldehyde | 0.15 | 0.02 | - | 0.01 | 0.01 | 0.02 |
| α-Pinene | 0.76 | 1.31 | 0.60 | 0.87 | 2.41 | 0.91 |
| 2,5-Diethyl THF | 0.08 | 0.03 | 0.09 | - | - | - |
| Camphene | 0.02 | 0.03 | - | - | - | - |
| β-Pinene | 0.65 | 1.08 | 0.65 | 1.23 | 8.70 | 1.22 |
| Sabinene | 0.43 | 0.76 | 0.51 | 0.59 | 1.39 | 0.66 |
| Myrcene | 2.61 | 3.32 | 3.36 | 0.29 | 1.49 | 0.27 |
| α-Phellandrene | 0.04 | 0.06 | - | - | - | - |
| α-Terpinene | 0.30 | 0.07 | 0.45 | 0.10 | 0.65 | 0.12 |
| 1-Limonene | 11.74 | 16.12 | 14.02 | 2.05 | 1.64 | 2.12 |
| 1, 8-Cineole | 1.71 | 1.40 | 1.82 | 3.58 | 2.40 | 4.62 |
| trans-Ocimene | - | - | - | - | 8.22 | - |
| cis-Ocimene | 0.21 | 0.42 | 0.37 | 0.46 | - | 0.23 |
| γ-Terpinene | 0.50 | 0.12 | 0.79 | 0.29 | 7.15 | 0.20 |
| p-Cymene | 0.05 | 0.01 | 0.13 | 0.07 | 2.56 | 0.10 |
| Terpinolene | 0.17 | 0.11 | 0.27 | 0.10 | 0.19 | 0.10 |
| 3-Octyl acetate | - | 0.22 | - | - | - | - |
| cis-3-Hexenol | 0.02 | 0.02 | - | - | - | - |
| 3-Octanol | 0.87 | 0.52 | - | 0.22 | 0.36 | 0.27 |
| 1-Octen-3-ol | - | - | - | 0.04 | 0.02 | 0.08 |
| trans-2-Hexenol | - | 0.02 | - | - | - | - |
| trans-sabinene hydrate | 0.88 | 1.98 | - | 1.23 | 0.07 | 2.02 |
| 1-Menthone | 0.12 | 0.10 | - | 14.09 | 0.16 | 21.70 |
| 2-Ethyl-1-hexanol | 0.01 | 0.00 | - | - | - | - |
| cis-3-Hexenyl isovalerate | 0.03 | 0.05 | 0.05 | - | - | - |
| Menthofuran | - | - | - | 7.09 | 0.32 | 7.22 |
| Isomenthone | 0.10 | 0.02 | - | 1.84 | - | 2.08 |
| β-Bourbonene | 1.43 | 1.91 | 0.82 | 0.29 | 0.07 | 0.15 |
| Linalool | - | 0.09 | - | 0.37 | 41.81 | 0.28 |
| cis-sabinene hydrate | - | - | - | 0.02 | - | 0.02 |
| Menthyl acetate | 0.07 | 0.04 | - | 9.16 | - | 7.97 |
| Isopulegol | 0.19 | 0.05 | - | 0.05 | - | 0.05 |
| Isopulegone | - | - | - | 0.50 | - | 0.45 |
| Neomenthol | 0.11 | 0.27 | - | 3.33 | - | 3.10 |
| Terpinen-4-ol | 0.93 | 0.23 | 1.13 | 0.22 | 0.11 | 0.34 |
| β-Caryophyllene | 0.83 | 0.58 | 0.92 | 1.84 | 5.77 | 1.76 |
| cis-Dihydrocarvone | 1.01 | 2.11 | 0.75 | - | - | - |
| trans- Dihydrocarvone | - | 0.03 | - | - | - | - |
| N eoisomenthol | - | - | - | 0.87 | - | 0.75 |
| 1-Menthol | 0.35 | 0.35 | - | 41.07 | - | 33.33 |
| Pulegone | 0.72 | 0.20 | - | 0.11 | - | 0.44 |
| Isomenthol | - | - | - | 0.24 | - | 0.22 |
| β-Farnesene | 0.36 | 1.08 | 1.09 | 0.54 | 0.16 | 0.60 |
| Dihydrocarvyl Acetate | 0.05 | 0.69 | - | - | - | - |
| β-Bisabolene | - | 0.17 | - | - | - | - |
| β-Cubebene | - | 0.11 | - | - | - | - |
| α-Humulene | - | - | - | 0.11 | 0.23 | 0.11 |
| α-Terpineol | 0.05 | 0.15 | 0.29 | 0.16 | 0.64 | 0.22 |
| Germacrene- D | 0.50 | 1.76 | 3.37 | 2.43 | 3.00 | 2.38 |
| Piperitone | 0.16 | 0.06 | 0.12 | 0.31 | 0.57 | 0.37 |
| 1-Carvone | 66.49 | 56.74 | 59.47 | 0.50 | 0.47 | - |
| Dihydrocarveol | 0.05 | 0.06 | 0.11 | - | - | - |
| Carvyl Acetate | 0.29 | 0.60 | 0.54 | - | - | - |
| Carveol I | 0.40 | 0.44 | 0.67 | - | - | - |
| Carvone Oxide | 0.09 | 0.13 | - | - | - | - |
| Carveol II | 0.26 | 0.27 | 0.23 | - | - | - |
| Piperitone oxide | - | - | - | - | 0.57 | - |
| cis-Jasmone | 0.28 | 0.31 | 0.41 | - | - | - |
| Viridiflorol | - | 0.35 | - | 0.45 | - | 0.34 |
| Thymol | - | - | - | - | 2.53 | 0.09 |
| Eugenol | - | - | - | - | 0.15 | - |
| Isoamyl phenylacetate | - | - | - | - | 0.67 | - |
| Carvacrol | - | - | - | - | 0.17 | - |

In addition to the differences in makeup of the oils, Table 7 compares the organoleptic properties of spearmint, peppermint and macho mint plant oils. Additionally, Table 8 shows the chiral constituents of scotch spearmint, native spearmint, Macho mint, Julep mint, and Ginger mints.

**Table 7. Organoleptic analysis of select Mentha essential oils**

| Native Spearmint | Macho Mint | Julep Mint | Ginger Mint |
|---|---|---|---|
| Fresh, carvone | Fresh, spearmint, leaf-like | Full, green, leaf-like | Spicy, floral, crisp |

**Table 8. Proportions of select chiral chemicals**

| Chiral Chemicals | Scotch Spearmint | Native Spearmint | Macho Mint | Julep Mint | Ginger Mint |
|---|---|---|---|---|---|
| (1S)-(-)-α-pinene | 68 | 68 | 66 | 66 | 66 |
| (1 R)-(+)-α-pinene | 32 | 32 | 34 | 32 | 32 |
| (+)-sabinene | 59 | 60 | 61 | 61 | 43 |
| (-)-sabinene | 41 | 40 | 39 | 39 | 57 |
| (+)-β-pinene | 54 | 56 | 53 | 40 | 68 |
| (-)-β-pinene | 46 | 44 | 47 | 60 | 32 |
| (S)-(-)-limonene | 99 | 99 | 99 | 99 | 99 |
| (R)-(+)-limonene | <1 | <1 | <1 | <1 | <1 |
| (3R)-(-)-linalool | - | - | - | - | 99 |

The presently disclosed mint oils can be extracted from their respective mint plants by a variety of methods known in the art including, but not limited to, steam distillation, vacuum distillation, supercritical gas and solvent extraction. Once extracted, the different mint oils can be incorporated into a consumer product in amounts ranging from about 0.01% to about 5.0%, from about 0.02% to about 4.0%, from about 0.02% to about 3.0%, from about 0.02% to about 2.0%, and from about 0.02% to about 1.0% by weight of the product. The mint oils can be used alone or in combination with other mint oils.

### 3. Additional Compounds

In certain embodiments of the disclosed subject matter, the various mint cultivar oils may be using in conjunction with additional compounds. In certain non-limiting embodiments, additional compounds include, but are not limited to, cooling agents, warming agents and additional flavor compounds.

Cooling agents are additives that provide a cooling or refreshing effect in the mouth, in the nasal cavity, or on the skin. Examples of cooling agents are menthol, N-ethyl-p-menthane-3-carboxamide [WS-3], N,2,3-trimethyl-2-isopropyl-butanamide [WS-23], ethyl 3-(p-menthane-3-carboxamido) acetate [WS-5], [(2-isopropyl-5-methyl-cyclohexanecarbonyl)- amino]-acetic acid isopropyl ester, menthyl glutarate, menthyl succinate, menthyl lactate, 3-1-menthoxypropane-1,2-diol, eucalyptol, menthone, isomenthone, and isopulegol, and combinations thereof. These are other suitable cooling agents that can be used in the oral product are further described in WO 2011/159935, which is incorporated herein by reference in its entirety.

The concentration of cooling agent in the oral product may be from 0.01% to 5.0% and more specifically may be from 0.02% to 2.0%. The cooling agent can also be from an essential oil that contains cooling compounds. For example, peppermint oil has menthol in it and therefore a combination of ginger mint oil and peppermint oil may also be used in an oral product (including chewing gum). In an embodiment, the cooling agent is added separately to the oral product and does not come from an essential oil (such as peppermint). In an embodiment, the cooling agent is something other than menthol.

The presently disclosed subject matter contemplates that optionally sensates such as warming agents and/or tingling agents may also be included in an oral product. Warming agents provide to the user a sensation of warmth and tingling agents provide to the user a sensation of tingling. Examples of warming and tingling agents can be found in U.S. Patent No. 6,890,567, which is incorporated herein by reference in its entirety.

A variety of flavoring agents can also be used in addition to the mint oils in various end products, if desired. The flavoring agent can be used in liquid or solid form. Flavoring agents may include artificial or natural flavors known in the art, for example synthetic flavor oils, natural flavoring aromatics and/or oils, oleoresins, extracts derived from plants, leaves, flowers, fruits, fruit flavors, and the like, or a combination thereof. Non-limiting representative flavors include oils such as spearmint oil (*mentha spicata*), cinnamon oil, oil of wintergreen (methyl salicylate), peppermint oil (*mentha piperita*), clove oil, bay oil, anise oil, eucalyptus oil, thyme oil, cedar leaf oil, oil of nutmeg, allspice, oil of sage, mace, oil of bitter almonds, cassia oil, and citrus oils including lemon, orange, lime, grapefruit, vanilla, fruit essences, including apple, pear, peach, grape, strawberry, raspberry, blackberry, cherry, plum, pineapple, apricot, banana, melon, tropical fruit, mango, mangosteen, pomegranate, papaya, honey lemon, and the like, or a combination thereof.

Artificial flavor components are also contemplated by the present invention. Those of ordinary skill in the art will recognize that natural and artificial flavors may be combined in any sensorially acceptable blend. All such flavors and blends are contemplated by the presently disclosed subject matter.

### 4. Flavor Compositions

The presently disclosed subject provides for different flavor compositions that include at least one, two, three or more mint cultivar oils. In a specific embodiment, the flavor composition includes one mint cultivar oil.

In certain non-limiting embodiments, the composition comprises one or more of ginger mint oil, blue balsam tea mint oil, macho mint oil, julep mint oil, banana mint oil, sweet pear mint oil and lavender mint oil, and further comprises spearmint oil and/or peppermint oil.

In certain non-limiting embodiments, the composition comprises a mixture of macho mint oil, julep mint oil, and peppermint oil.

In certain non-limiting embodiments, the composition comprises ginger mint oil, lavender mint oil, and peppermint oil.

In certain non-limiting embodiments, the composition comprises a mixture of peppermint oil and sweet pear mint oil.

In certain non-limiting embodiments, the composition comprises a mixture of peppermint oil and ginger mint oil.

In certain non-limiting embodiments, the composition comprises lavender mint oil and peppermint oil.

In certain non-limiting embodiments, the composition comprises a mixture of peppermint oil and banana mint oil.

In certain non-limiting embodiments, the one or more mint cultivar oils is present in amounts effective to provide a primary sensory note selected from the group consisting of spearmint, peppermint, or combinations thereof.

In certain non-limiting embodiments, the one or more mint cultivar oils is present in amounts effective to provide a secondary sensory note selected from the group consisting of vanilla, peppery, chalky, green, leafy, piney, fish oil, citrus, lemon, creamy, woody, herbal, lemon pith, plasticy, metallic, buttermint, spicy, honey, green tea, lemon zest and combinations thereof.

In non-limiting embodiments, the composition comprises ginger mint oil, blue balsam tea mint oil, macho mint oil, julep mint oil, banana mint oil, sweet pear mint oil, lavender mint oil, spearmint oil, and/or peppermint oil in an amount per oil of about 0.01 to about 5 % by weight, about 0.02 to about 2.0 % by weight, or about 0.02 to about 0.2 % by weight of the total composition.

In certain non-limiting embodiments, the oral composition comprises 0.1-5.0 % peppermint oil and about 0.1-3.0% sweet pear mint oil by weight. In certain non-limiting embodiments, the oral composition comprises about 0.1-5.0 % peppermint oil and about 0.1-3.0% ginger mint oil by weight. In certain non-limiting embodiments, the oral composition comprises about 0.1-5.0 % peppermint oil and about 0.01-1.0% banana mint oil by weight. In certain non-limiting embodiments, the oral composition comprises about 0.1-5.0 % peppermint oil, 0.1-3.0% macho mint oil and about 0.1-3.0% sweet pear mint oil by weight. In certain non-limiting embodiments, the oral composition comprises about 0.1-5.0 % peppermint oil, 0.05-2.0 % ginger mint oil and about 0.01-1.0% lavender mint oil by weight. In certain non-limiting embodiments, the oral composition comprises about 0.1-5.0 % peppermint oil and about 0.01-1.0% lavender mint oil by weight.

In certain embodiments of the present application, the mint cultivar oil is encapsulated prior to use in an end product. In certain embodiments, the encapsulated formulation is admixed with a food product wherein the encapsulated mint oil is present in an amount of from about 0.01 to about 5%, or from about 0.05 to about 2.5%, or from about 0.1 to about 2%, or from about 0.2 to about 1%, or from about 0.25 to about 8%, or from about 0.25 to about 0.75%, or from about 0.25 to about 0.7%, and values in between.

In certain non-limiting embodiments, the oral composition further comprises one or more cooling agents from about 0.01% to about 5.0% or from about 0.02% to about 2.0%.

In certain embodiments, the mint cultivar oils can be used to enhance or increase a sensory attribute of an oral composition, such as chewing gum or compressed mints. In further embodiments, the mint cultivar oils provide an enhanced breath freshening sensory attribute in the composition. In certain embodiments, the composition comprises two or more mint cultivar oils that provide a greater than additive (*i.e*., synergistic) enhancement of a sensory attribute when admixed with an edible composition, such as food compositions.

The mint cultivar oils of the presently disclosed subject matter can be used to enhance or modify flavor duration, breath freshening effect and overall consumer liking scores of an edible composition. The compositions can include combinations of mint cultivar oils, and can be added to edible compositions in different consumer products. Additionally, one or more flavor compositions can be added to different consumer products.

### 5. Consumer Products

One or more of the flavor compositions of the disclosed subject matter can be used to enhance or modify a sensory attribute of various edible food products including but not limited to oral products, such as chewing gum, mints, confections or candies, lozenges, edible film, pastilles, dentifrice, toothpaste, mouthwash, mouth spray, tobacco, and pharmaceuticals.

The concentration of a flavor composition admixed with an edible food product to modulate or enhance a sensory attribute of the food product can vary depending on the specific type of edible product. In certain embodiments, the flavor composition is admixed with a food product in an amount effective to enhance a sensory attribute by about 1 to about 100 fold, or from about 1 to about 50 fold, or from about 1 to about 10 fold, or from about 1.25 to about 8 fold, or from about 1.5 to about 6 fold, or from about 1.75 to about 4 fold, or from about 2 to about 2.5 fold, and values in between, compared to a sensory attribute reference.

In certain embodiments, the flavor compositions of the present application can be incorporated into a delivery system for use in edible products. Delivery systems can be liquid or solid, aqueous or non-aqueous. Delivery systems are generally adapted to suit the needs of the mint cultivar oils, flavor compositions, and/or the edible composition into which the mint cultivar oils will be incorporated.

### 5.1 Chewing Gum

In a specific embodiment, the consumer product is a chewing gum product. A chewing gum product comprises a gum base, mint compound, and a cooling agent. In general, a chewing gum composition typically contain a chewable gum base portion which is essentially free of water and is water-insoluble, a water-soluble bulk portion and flavors which are typically water insoluble. The water-soluble portion dissipates with a portion of the flavor over a period of time during chewing. The gum base portion is retained in the mouth throughout the chew.

The insoluble gum base generally comprises elastomers, elastomer solvents, plasticizers, waxes, emulsifiers and inorganic fillers. Plastic polymers, such as polyvinyl acetate, which behave somewhat as plasticizers, are also often included. Other plastic polymers that may be used include polyvinyl laureate, polyvinyl alcohol and polyvinyl pyrrolidone.

Elastomers may include polyisobutylene, butyl rubber, (isobutylene-isoprene copolymer) and styrene butadiene rubber, as well as natural latexes such as chicle. Elastomer solvents are often resins such as terpene resins and rosin esters. Plasticizers, sometimes called softeners, are typically fats and oils, including tallow, hydrogenated and partially hydrogenated vegetable oils, and cocoa butter. Commonly employed waxes include paraffin, microcrystalline and natural waxes such as beeswax and carnauba. Microcrystalline waxes, especially those with a high degree of crystallinity, may be considered bodying agents or textural modifiers.

According to the preferred embodiment of the present invention, the insoluble gum base constitutes between about 5% to about 95% by weight of the gum. More preferably the insoluble gum base comprises between 10% and 50% by weight of the gum and most preferably about 20% to 35% by weight of the gum.

The gum base typically also includes a filler component. The filler component may be calcium carbonate, magnesium carbonate, talc, dicalcium phosphate or the like. The filler may constitute between about 5% and about 60% by weight of the gum base. Preferably the filler comprises about 5% to 50% by weight of the gum base.

Gum bases typically also contain softeners including glycerol monostearate and glycerol triacetate. Gum bases may also contain optional ingredients such as antioxidants, colors, and emulsifiers. The present invention contemplates employing any commercially acceptable gum base.

The water-soluble portion of the chewing gum may further comprise softeners, sweeteners, flavoring agents, fillers, cooling agents and combinations thereof. The sweeteners often fulfill the role of bulking agents in the gum. The bulking agents typically comprise about 5% to about 95% of the gum composition.

Softeners are added to the chewing gum in order to optimize the chewability and mouth feel of the gum. Softeners, also known in the art as plasticizers or plasticizing agents, generally constitute between about 0.5% to about 15% of the chewing gum. Softeners contemplated by the present invention include glycerin, lecithin and combinations thereof. Further, aqueous sweetener solutions such as those containing sorbitol, hydrogenated starch hydrolysate, corn syrup and combinations thereof may be used as softeners and binding agents in gum.

As mentioned above, the presently disclosed mint cultivar oils and cooling agents may be used in sugar and sugarless gum formulations. Sugar sweeteners generally include saccharide-containing components commonly known in the chewing gum art which comprise, but are not limited to, sucrose, dextrose, maltose, dextrin, dried invert sugar, fructose, galactose, corn syrup solids and the like, alone or in any combination. Sugarless sweeteners include components with sweetening characteristics but which are devoid of the commonly known sugars and comprise, but are not limited to, sugar alcohols such as sorbitol, hydrogenated isomaltulose, mannitol, xylitol, lactitol, erythritol, hydrogenated starch hydrolysate, maltitol and the like alone or in any combination.

Depending on the particular sweetness release profile and shelf-stability needed, free or encapsulated high-intensity sweeteners may be used in the chewing gum composition, or may be used in a coating applied to centers made from those gum compositions. High-intensity sweeteners may be used at levels from about 0.01% to about 3.0%. High-intensity sweeteners may be encapsulated or be used in neat for or a combination of neat and encapsulated. High intensity sweeteners that may be used in the chewing gum are: aspartame, saccharin, Thaumatin, alitame, saccharin salts, sucralose, Stevia, steviol glycosides, monellin, neotame, brazzein, monatin, and acesulfame K. Overall, the chewing gum composition will preferable comprise about 0.5% to about 90% sweetening agents. Most typically the sweetening agents will comprises at least one bulk sweetener and at least one high-intensity sweetener.

Optional ingredients such as colors, emulsifiers and pharmaceutical agents may also be added as separate components of the chewing gum composition, or added as part of the gum base.

Aqueous syrups, such as corn syrup and hydrogenated corn syrup may be used, particularly if their moisture content is reduced. This can preferably be done by coevaporating the aqueous syrup with a plasticizer, such as glycerin or propylene glycol, to a moisture content of less than 10%. Preferred compositions include hydrogenated starch hydrolysate solids and glycerin. Such syrups and their methods of preparation are discussed in detail in U.S. Patent No. 4,671,967.

A preferred method of manufacturing chewing gum according to the present invention is by sequentially adding the various chewing gum ingredients to any commercially available mixer known in the art. After the ingredients have been thoroughly mixed, the gum is discharged from the mixer and shaped into the desired form such as by rolling into sheets and cutting into sticks, extruding into chunks, or casting into pellets.

Generally, the ingredients are mixed by first melting the gum base and adding it to the running mixer. The base may also be melted in the mixer itself. Color or emulsifiers may also be added at this time, along with syrup and a portion of the bulking agent. Further portions of the bulking agent may then be added to the mixer. A flavoring agent, e.g., ginger mint, may be added with the final portion of the bulking agent. The cooling agent may be mixed with the flavor composition of the present invention and preferably added as part of the flavor addition. If the mint or cooling agent is encapsulated to modify its release rate, it will preferably be added after the final portion of bulking agent and flavor has been added. The entire mixing procedure typically takes from five to twenty minutes, but longer mixing times may sometime be required. Those skilled in the art will recognize that many variations of the above described procedures may be followed.

If formed into pellets or balls, the chewing gum composition can be coated. The coating is initially present as a liquid syrup which contains from about 30% to about 80% or 85% sugars or sugar alcohols, and from about 15% or 20% to about 70% of a solvent such as water. In general, the coating process is carried out in conventional panning equipment. Gum center tablets to be coated are placed into the panning equipment to form a moving mass.

The material or syrup which will eventually form the coating is applied or distributed over the gum center tablets. Flavors may be added before, during and after applying the syrup to the gum centers. Once the coating has dried to form a hard surface, additional syrup additions can be made to produce a plurality of coatings or multiple layers of coating.

In the panning procedure, syrup is added to the gum center tablets at a temperature range of from about 100°F to about 240°F. Preferably, the syrup temperature is from about 140°F to about 200°F. Most preferably, the syrup temperature should be kept constant throughout the process in order to prevent the polyol in the syrup from crystallizing. The syrup may be mixed with, sprayed upon, poured over, or added to the gum center tablets in any way known to those skilled in the art.

In another embodiment, a soft coating is formed by adding a powder coating after a liquid coating. The powder coating may include natural carbohydrate gum hydrolysates, maltodextrin, gelatin, cellulose derivatives, starches, modified starches, sugars, sugar alcohols, natural carbohydrate gums and fillers like talc and calcium carbonate.

Each component of the coating on the gum center may be applied in a single layer or in a plurality of layers. In general, a plurality of layers is obtained by applying single coats, allowing the layers to dry, and then repeating the process. The amount of solids added by each coating step depends chiefly on the concentration of the coating syrup. Any number of coats may be applied to the gum center tablet. Preferably, no more than about 75 coats are applied to the gum center. More preferably, less than about 60 coats are applied and most preferably, about 30 to about 60 coats are applied. In any event, the present invention contemplates applying an amount of syrup sufficient to yield a coated chewing gum product containing about 10% to about 65% coating. Preferably, the final product will contain from about 20% to about 50% coating.

Those skilled in the art will recognize that in order to obtain a plurality of coated layers, a plurality of premeasured aliquots of coating syrup may be applied to the gum center. It is contemplated, however, that the volume of aliquots of syrup applied to the gum center may vary throughout the coating procedure.

Once a coating of syrup is applied to the gum center, the syrup is dried in an inert medium. A preferred drying medium comprises air. Preferably, forced drying air contacts the wet syrup coating in a temperature range of from about 70°F to about 110°F. More preferably, the drying air is in the temperature range of from about 80°F to about 100°F. The invention also contemplates that the drying air possesses a relative humidity of less than about 15 percent. Preferably, the relative humidity of the drying air is less than about 8 percent.

The drying air may be passed over and admixed with the syrup coated gum centers in any way commonly known in the art. Preferably, the drying air is blown over and around the syrup coated gum center. If a flavor is applied after a syrup coating has been dried, the present invention contemplates drying the flavor with or without the use of a drying medium.

The mint oil, e.g., ginger mint oil, may also be fully or partially encapsulated with water-soluble or water-insoluble materials. Some encapsulation procedures include spray drying, spray chilling, fluid-bed coating, coacervation, extrusion, and other agglomerating and encapsulating techniques. Encapsulation materials include acrylic polymers and copolymers, carboxyvinyl polymer, polyamides, polystyrene, polyvinyl acetate, polyvinyl acetate phthalate, polyvinyl pyrrolidone, waxes, shellac, zein, agar, alginates, a wide variety of cellulose derivatives like ethyl cellulose and hydroxypropylmethyl cellulose, dextrin, gelatin, modified starches, acacia, maltodextrin, gum arabic, guar gums, locust bean gum, carrageenan, and mixtures thereof. Additionally, the mint oil may also be adsorbed onto an inert or water-insoluble material such as silicas, silicates, pharmasorb clay, sponge-like beads or microbeads, amorphous carbonates and hydroxides, including aluminum and calcium lakes. The mint oil may be modified in a multiple step process comprising any of the techniques noted.

### 5.2 Oral Products

As noted above, the presently disclosed subject matter can be incorporated into additional consumer products including, for example, food and confectionery products.

In certain embodiments, mint cultivar oils of the present disclosure can be incorporated into a confectionery product by admixing one or more mint cultivar oils or one or more flavor compositions into conventional hard and soft confections. In certain embodiments, the confectionery products include, but are not limited to, cakes, cookies, pies, candies (hard and soft), compressed mints, chewing gums, gelatins, ice creams, sorbets, jams, jellies, chocolates, fudge, fondant, liquorice, and taffy.

The presently disclosed subject matter can be incorporated into mint confectioneries, and more particularly into compressed mint products using conventional tablet pressing procedures and equipment and suitable additional components known in the art, for example, as described by U.S. Patent No. 8,557,323 and U.S. Patent No. 8,431,150, each of which is incorporated by reference in its entirety herein.

The presently disclosed subject matter can also be incorporated into oral products such as, but not limited to, lozenge, edible film, pastilles, dentifrice, toothpaste, mouthwash, mouth spray, tobacco, pharmaceuticals, and edible films.

Oral products of the presently disclosed subject matter can contain sugar or can be sugarfree. In addition to the disclosed mint cultivar oils and flavor compositions, other suitable flavoring agents can be included. Additional ingredients that give a tingling sensation can be included. In the case of products with multiple layers, each layer may have different flavoring agents or levels. In one embodiment, the compressed mint can comprise a coating layer covering at least a portion of the product. In that case, the coating layer can contain flavoring agents at a level higher than any flavoring agents in the remainder of the product.

The oral products can include one or more anti-microbial agents; physiological cooling agents; breath freshening agents; breath freshening and mouth odor masking flavors; dental active agents; and combinations thereof.

Anti-microbial agents include but are not limited to cardamom oil, magnolia bark extract, cranberry, geraniol, cinnamaldehyde, peppermint, triclosan, chlorhexidine, cetyl pyridinium chloride (CPC) and mixtures thereof.

Physiological cooling agents include menthol N-2,3-trimethyl-2-isopropyl butanamide, 3-1-menthoxypropane-1,2-diol, N-ethyl-p-menthane-3-carboxamide, menthane ketals, menthyl succinate, isopulegol, menthyl glutarate, and mixtures thereof.

Breath freshening agents include but are not limited to salts of zinc, salts of copper, polyphenols, mushroom extracts and mixtures thereof.

Breath freshening and mouth odor masking flavors include but are not limited to cinnamon, mint, wintergreen, fruit flavors and mixtures thereof.

Dental active agents include but are not limited to tooth whiteners, fluoride, stain removers, calcium salts, phosphate salts and mixtures thereof.

### EXAMPLES

The presently disclosed subject matter will be better understood by reference to the following Examples, which are provided as exemplary of the disclosed subject matter, and not by way of limitation.

### Examples 1 and 2: Gum formulations containing new cultivars

These Examples provide specific examples of chewing gum formulations with ginger mint oil.

**Table 9. Ginger Mint Chewing Gum Formulations**

| | Example 1 | Example 2 |
|---|---|---|
| ITEM NAME | % | % |
| Sorbitol powder | 53.14 | 53.14 |
| Gum base | 6.35 | 6.35 |
| Peppermint Oil | 0 | 0.56 |
| Ginger mint oil | 1.11 | 0.56 |
| Menthol | 0.94 | 0.94 |
| Liquid flavor | 0.56 | 0.56 |
| HSH glycerine blend | 35.73 | 35.73 |
| Glycerol | 0.80 | 0.80 |
| High intensity sweetener | 0.97 | 0.97 |
| Spray-dried flavor | 0.25 | 0.25 |
| Color | 0.10 | 0.10 |
| Salt solution | 0.05 | 0.05 |
| Total | 100 | 100 |

### Example 3: Flavor blend formulations containing new mint oils

This Example provides examples of certain flavor blends that can be incorporated into an oral product (including a chewing gum product).

**Table 10. Flavor blend formulations.**

| Item Name | Flavor Blend 3A | Flavor Blend 3B | Flavor Blend 3C | Flavor Blend 3D | Flavor Blend 3E | Flavor Blend 3F | Flavor Blend 3G |
|---|---|---|---|---|---|---|---|
| Ginger mint oil | 50 | 15 | 45 | 20 | 25 | 65 | 75 |
| Peppermint oil | - | 25 | - | 30 | - | - | - |
| Spearmint oil | 30 | - | 25 | - | - | - | - |
| Coolant A | 5 | - | - | 5 | - | 23 | 25 |
| Coolant B | - | 5 | - | - | 5 | - | - |
| Menthol | 10 | 40 | 10 | 30 | 50 | 12 | - |
| Eucalyptol | 5 | 5 | 10 | 10 | - | - | - |
| Coolant C | - | 5 | 10 | - | 10 | - | - |
| Coolant D | - | 5 | - | 5 | 10 | - | - |
| Total % | 100 | 100 | 100 | 100 | 100 | 100 | 100 |

### Example 4: Flavor formulations containing new cultivars

In this example, a variety of the mint cultivars of the disclosed subject matter were used to flavor a gum composition. The flavor formulations are summarized in Table 11. The gum formulas are similar to those used in Table 9.

**Table 11. Flavor Formulations (ingredients listed in percentage).**

| Formula/Ingredient | Sweet Pear Mint Flavor Formula % | Ginger Mint Flavor Formula % | Banana Mint Flavor Formula % | Macho Mint & Julep Mint Flavor Formula % | Ginger Mint & Lavender Mint Flavor Formula % | Lavender Mint Flavor Formula % |
|---|---|---|---|---|---|---|
| Peppermint oil | 20.42 | 23.36 | 34.89 | 21.65 | 25.64 | 34.89 |
| Dementholized Arvensis Oil | 4.14 | 4.23 | 4.15 | 4.15 | 4.17 | 4.15 |
| Coolant A | 9.31 | 9.53 | 9.35 | 9.35 | 9.38 | 9.35 |
| Coolant B | 3.73 | 3.81 | 3.74 | 3.74 | 3.75 | 3.74 |
| Menthol | 35.39 | 36.20 | 35.53 | 35.53 | 35.66 | 35.53 |
| Coolant C | 3.73 | 3.81 | 3.74 | 3.74 | 3.75 | 3.74 |
| Coolant D | 5.96 | 3.81 | 5.61 | 5.61 | 5.26 | 5.61 |
| Sweet Pear Mint | 17.32 | | | | | |
| Ginger Mint | | 15.24 | | | 9.38 | |
| Banana Mint | | | 2.99 | | | |
| Macho Mint | | | | 5.42 | | |
| Julep Mint | | | | 10.81 | | |
| Lavender Mint | | | | | 3.00 | 2.99 |
| Total | 100.00 | 100.00 | 100.00 | 100.00 | 100.00 | 100.00 |

Each gum formulation was chewed and rated by consumers (n=191) on a scale of 0-9 for a variety of attributes including effectiveness of breath freshening and overall liking.

It was unexpectedly found that lavender mint and macho/julep mints achieved breath freshening similar to the peppermint control as rated on the scale of 0-9, as indicated in Table 12 below.

**Table 12. Ratings of chewing gum formulations for breath freshening attributes.**

| | Peppermint | Spearmint | Sweet pear mint | Ginger mint | Banana mint | Macho & Julep mint | Ginger & Lavender mint | Lavender mint |
|---|---|---|---|---|---|---|---|---|
| Mean | 7.14 | 6.96 | 6.85 | 6.66 | 6.62 | 7.09 | 6.96 | 7.10 |

It was also found that a mixture of macho mint and julep mint resulted in similar overall liking as the spearmint control, as rated on a scale of 0-9 as indicated in Table 13 below.

**Table 13. Ratings of chewing gum formulations for overall liking.**

| | Peppermint | Spearmint | Sweet pear mint | Ginger mint | Banana mint | Macho & Julep mint | Ginger & Lavender mint | Lavender mint |
|---|---|---|---|---|---|---|---|---|
| Mean | 7.19 | 7.12 | 6.61 | 6.43 | 6.22 | 6.97 | 6.86 | 6.85 |

### Example 5: Descriptive analysis of chewing gum formulations.

In this example, nine trained descriptive analysis (DA) panelists described the primary and secondary notes detected from a number of gum formulations comprising the disclosed mint cultivar oils. The panelists suggested corresponding names for each chewing gum formulation based on the notes detected. The results are summarized in Table 14.

**Table 14. Descriptive analysis of gum formulations.**

| SAMPLE | **Control** Peppermint Stick | **Control** Spearmint Stick | Sweet Pear Mint Gum | Ginger Mint Gum | Banana Mint Gum | Macho Mint & Julep Mint Gum | Ginger Mint & Lavender Mint Gum | Lavender Mint Gum |
|---|---|---|---|---|---|---|---|---|
| PRIMARY NOTES | Peppermint | Spearmint | Peppermint | Peppermint | Peppermint | Spearmint | Peppermint | Peppermint |
| SECONDARY NOTES | Buttermint | Green/Leafy | Peppery/Spicy | Green Tea | Green | Peppermint | Citrus (Lemon) | Peppery |
| | Vanilla | Piney | Herbal | Citrus (Lemon) | Metallic | Piney | Lemon Zest | Spicy |
| | Peppery | Vanilla | Metallic | Honey | | Green/Leafy | Lemon Pith | Creamy |
| | Creamy | Creamy | Fish Oil | | | Peppery | Peppery | |
| | | | Buttermint | | | | | |

A number of the secondary notes were found to be surprising and unexpected.

### Example 6: Gum formulations containing a mixture of cultivars

In this example, a variety of the disclosed mint cultivars were combined and used to flavor a gum composition. Six gum compositions made using the same gum formulas as Table 9 with the following mixture of mint cultivar oils are summarized in Table 15.

**Table 15. Flavor compositions.**

| P3 | P4 | P5 | P6 | P7 | P8 |
|---|---|---|---|---|---|
| Peppermint & Sweet Pear Mint | Peppermint & Ginger Mint | Peppermint & Banana Mint | Peppermint & Macho Mint & Julep Mint | Peppermint & Ginger Mint & Lavender Mint | Peppermint & Lavender Mint |

Each formulation was chewed and rated by consumers (n=191) on a scale of 0-9 for a variety of attributes including effectiveness of breath freshening and overall liking. The results are summarized in Table 16. The rankings for each formulation were recorded and compared to a spearmint and peppermint control. It was surprisingly found that formulations P6, P7, and P8 were comparable to the control formulations in both effectiveness and overall flavor lastingness.

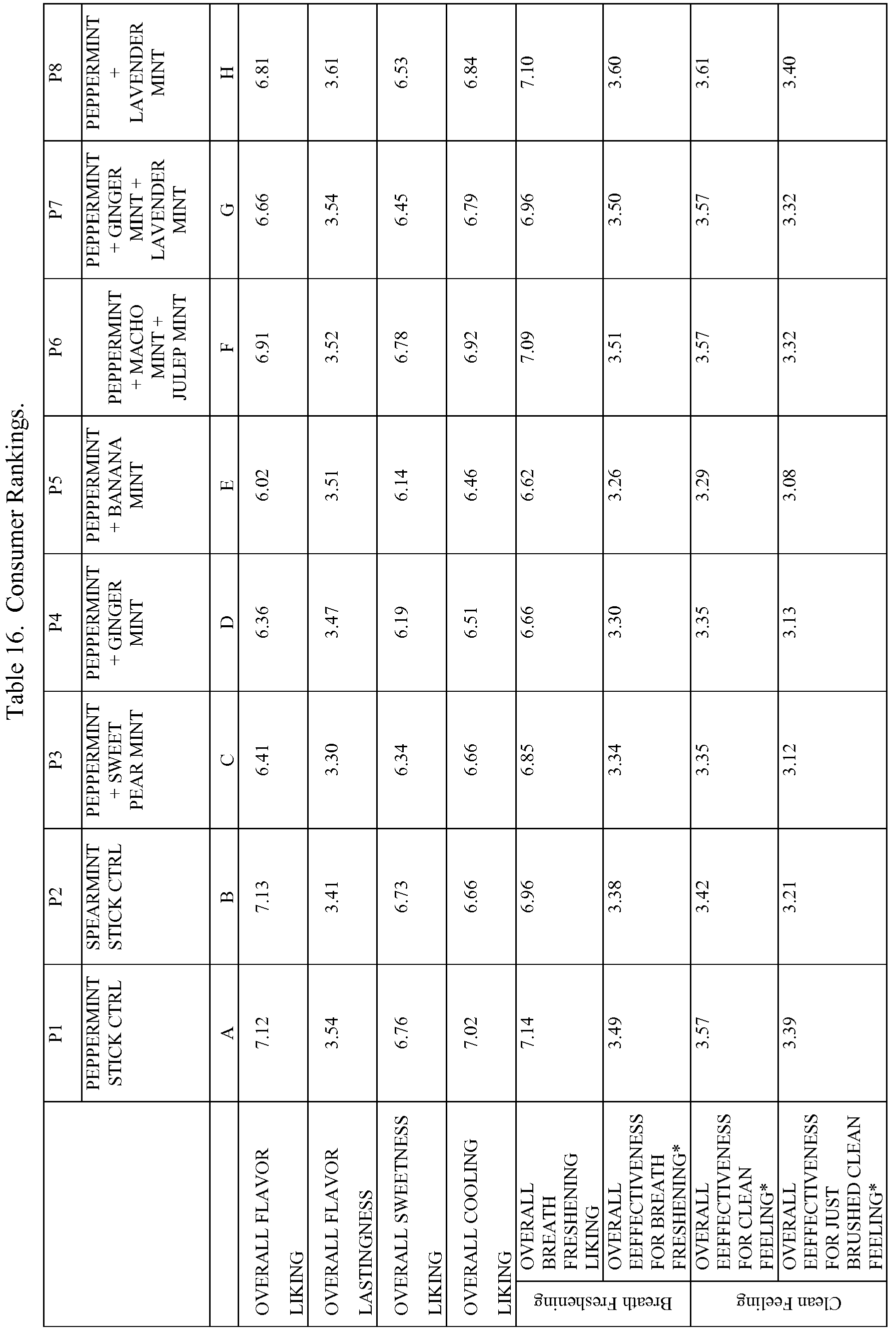

Although the presently disclosed subject matter and its advantages have been described in exemplary embodiments, it should be understood that various changes, substitutions and alterations can be made herein without departing from the scope of the disclosed subject matter as defined by the appended claims. In addition, many modifications can be made to adapt a particular situation or material to the teachings of the invention without departing from the essential scope thereof. Moreover, the scope of the present application is not intended to be limited to the particular embodiments of the process, machine, manufacture, composition of matter, means, methods and steps described in the specification. As one of ordinary skill in the art will readily appreciate from the disclosure of the presently disclosed subject matter, processes, machines, manufacture, compositions of matter, means, methods, or steps, presently existing or later to be developed that perform substantially the same function or achieve substantially the same result as the corresponding embodiments described herein may be utilized according to the presently disclosed subject matter. Accordingly, the appended claims are intended to include within their scope such processes, machines, manufacture, compositions of matter, means, methods, or steps.

## Claims

1. A composition comprising:
one or more mint cultivar oils selected from the group consisting of ginger mint, blue balsam tea mint, macho mint, julep mint, banana mint, sweet pear mint, lavender mint, and combinations thereof,
wherein the one or more mint cultivar oils is present in an amount of from about 0.01% to about 5.0% by weight of the total composition; and wherein the one or more mint cultivar oils is in an amount effective to provide a primary sensory note selected from the group consisting of spearmint, peppermint, and combinations thereof; and
wherein the composition further comprises peppermint and/or spearmint.

2. The composition of claim 1, wherein the one or more mint cultivar oils comprises ginger mint.

3. The composition of claim 1, wherein the one or more mint cultivar oils comprises blue balsam tea mint.

4. The composition of claim 1, wherein the one or more mint cultivar oils comprises macho mint.

5. The composition of claim 1, wherein the one or more mint cultivar oils comprises julep mint.

6. The composition of claim 1, wherein the one or more mint cultivar oils comprises banana mint.

7. The composition of claim 1, wherein one or more mint cultivar oils comprises sweet pear mint.

8. The composition of claim 1, wherein one or more mint cultivar oils comprises lavender mint.

9. The composition of claim 1, wherein the one or more mint cultivar oils comprises macho mint, julep mint, and combinations thereof, wherein the composition further comprises peppermint.

10. The composition of claim 1, wherein one or more mint cultivar oils comprises ginger mint, lavender mint, and combinations thereof, wherein the composition further comprises peppermint

11. The composition of any one of the preceding claims, which further comprises a cooling agent selected from the group consisting of menthol, N-ethyl-p-menthane-3-carboxamide, N,2,3- trimethyl-2-isopropyl-butanamide, ethyl 3-(p-menthane-3-carboxamido) acetate, [(2-isopropyl-5-methyl-cyclohexanecarbonyl)-amino]-acetic acid isopropyl ester, menthyl glutarate, menthyl succinate, menthyl lactate, 3-1-menthoxypropane-I,2-diol, eucaplyptol, and isopulegol, WS-5, WS-23 and combinations thereof and wherein the composition comprises between 0.01 % to 5.0% cooling agent by weight of the composition.

12. The composition of claim 11, wherein the composition comprises between 0.02% to 2.0% cooling agent by weight of the composition.

13. The composition of any one of the preceding claims, wherein the composition is formulated into a consumer product selected from the group consisting of chewing gum, confection, lozenge, pastilles, dentifrice, toothpaste, mouthwash, mouth spray, tobacco, pharmaceuticals, and edible films.

14. The composition of any one of the preceding claims, wherein the one or more mint cultivar oils is encapsulated.

15. The composition of any one of the preceding claims, further comprising a fruit flavor.

16. The composition of any one of the preceding claims, further comprising a sweetener.

17. The composition of any one of the preceding claims, where the one or more mint cultivar oils is present in an amount effective to provide a secondary sensory note selected from the group consisting of vanilla, peppery, chalky, green, leafy, piney, fish oil, citrus, lemon, creamy, woody, herbal, lemon pith, plasticy, metallic, buttermint, spicy, honey, green tea, lemon zest and combinations thereof.

## Patentansprüche

1. Zusammensetzung, umfassend:
ein oder mehrere Minzecultivar-Öle ausgewählt aus der Gruppe bestehend aus Ingwerminze, Blaue-Balsam-Teeminze, Macho-Minze, Julep-Minze, Bananenminze, süße Birnenminze, Lavendelminze und Kombinationen davon,
wobei das eine oder die mehreren Minzecultivar-Öle in einer Menge von etwa 0,01 Gew.-% bis etwa 5,0 Gew.-% der Gesamtzusammensetzung vorhanden ist; und wobei das eine oder die mehreren Minzecultivar-Öle in einer Menge vorhanden ist, die wirksam ist, eine primäre sensorische Note ausgewählt aus der Gruppe bestehend aus grüner Minze, Pfefferminze und Kombinationen davon zu verleihen; und
wobei die Zusammensetzung ferner Pfefferminze und/oder grüne Minze.

2. Zusammensetzung gemäß Anspruch 1, wobei das eine oder die mehreren Minzecultivar-Öle Ingwerminze umfasst.

3. Zusammensetzung gemäß Anspruch 1, wobei das eine oder die mehreren Minzecultivar-Öle Blaue-Balsam-Teeminze umfasst.

4. Zusammensetzung gemäß Anspruch 1, wobei das eine oder die mehreren Minzecultivar-Öle Macho-Minze umfasst.

5. Zusammensetzung gemäß Anspruch 1, wobei das eine oder die mehreren Minzecultivar-Öle Julep-Minze umfasst.

6. Zusammensetzung gemäß Anspruch 1, wobei das eine oder die mehreren Minzecultivar-Öle Bananenminze umfasst.

7. Zusammensetzung gemäß Anspruch 1, wobei das eine oder die mehreren Minzecultivar-Öle süße Birnenminze umfasst.

8. Zusammensetzung gemäß Anspruch 1, wobei das eine oder die mehreren Minzecultivar-Öle Lavendelminze umfasst.

9. Zusammensetzung gemäß Anspruch 1, wobei das eine oder die mehreren Minzecultivar-Öle Macho-Minze, Julep-Minze und Kombinationen umfasst, wobei die Zusammensetzung ferner Pfefferminze umfasst.

10. Zusammensetzung gemäß Anspruch 1, wobei das eine oder die mehreren Minzecultivar-Öle Ingwerminze, Lavendelminze und Kombinationen umfasst, wobei die Zusammensetzung ferner Pfefferminze umfasst.

11. Zusammensetzung gemäß einem der vorstehenden Ansprüche, ferner umfassend ein kühlendes Mittel ausgewählt aus der Gruppe bestehend aus Menthol, N-Ethyl-p-menthan-3-carboxamid, N-2,3-Trimethyl-2-isopropylbutanamid, Ethyl-3-(p-menthan-3-carboxamido)acetat, [(2-Isopropyl-5-methylcyclohexancarbonyl)amino]-essigsäureisopropylester, Menthylglutarat, Menthylsuccinat, Menthyllactat, 3-1-Menthoxypropan-1,2-diol, Eucalyptol und Isopulegol, WS-5, WS-23 und Kombinationen davon, und wobei die Zusammensetzung zwischen 0,01 Gew.-% und 5,0 Gew.-% an kühlendem Mittel, bezogen auf das Gewicht der Zusammensetzung, umfasst.

12. Zusammensetzung gemäß Anspruch 11, wobei die Zusammensetzung zwischen 0,02 Gew.-% und 2,0 Gew.-% an kühlendem Mittel, bezogen auf das Gewicht der Zusammensetzung, umfasst.

13. Zusammensetzung gemäß einem der vorstehenden Ansprüche, wobei die Zusammensetzung in ein Verbraucherprodukt ausgewählt aus der Gruppe bestehend aus Kaugummi, Konfekt, Lutschtablette, Pastillen, Zahnputzmittel, Zahnpasta, Mundspülung, Mundspray, Tabak, Pharmazeutika und essbare Filme formuliert ist.

14. Zusammensetzung gemäß einem der vorstehenden Ansprüche, wobei das eine oder die mehreren Minzecultivar-Öle verkapselt ist.

15. Zusammensetzung gemäß einem der vorstehenden Ansprüche, ferner umfassend ein Fruchtaroma.

16. Zusammensetzung gemäß einem der vorstehenden Ansprüche, ferner umfassend einen Süßstoff.

17. Zusammensetzung gemäß einem der vorstehenden Ansprüche, wobei das eine oder die mehreren Minzecultivar-Öle in einer Menge vorliegt, die wirksam ist, eine sekundäre sensorische Note ausgewählt aus der Gruppe bestehend aus Vanille, pfefferig, kalkig, grün, blättrig, kiefernartig, Fischöl, Citrus, Zitrone, sahnig, holzig, kräuterartig, Zitronenmark, kunststoffartig, metallisch, Buttermilch, würzig, Honig, Grüntee, Zitronenschale und Kombinationen davon zu verleihen.

## Revendications

1. Composition, comprenant :
une ou plusieurs huiles de cultivar de menthe choisies dans le groupe constitué par la menthe « Gingembre », la menthe balsamique bleue, la menthe « Macho », la menthe Julep, la menthe « Banane », la menthe « Poire Sucrée », la menthe « Lavande », et des combinaisons de celles-ci, dans laquelle l'huile ou les huiles de cultivar de menthe sont présentes en une quantité allant d'environ 0,01 % à environ 5,0 % en poids de la composition totale ; et dans laquelle l'huile ou les huiles de cultivar de menthe sont présentes en une quantité efficace pour apporter une note sensorielle primaire choisie dans le groupe constitué par la menthe verte, la menthe poivrée, et des combinaisons de celles-ci ; et
la composition comprenant en outre de la menthe poivrée et/ou de la menthe verte.

2. Composition selon la revendication 1, dans laquelle l'huile ou les huiles de cultivar de menthe comprennent de la menthe « Gingembre ».

3. Composition selon la revendication 1, dans laquelle l'huile ou les huiles de cultivar de menthe comprennent de la menthe balsamique bleue.

4. Composition selon la revendication 1, dans laquelle l'huile ou les huiles de cultivar de menthe comprennent de la menthe « Macho ».

5. Composition selon la revendication 1, dans laquelle l'huile ou les huiles de cultivar de menthe comprennent de la menthe Julep.

6. Composition selon la revendication 1, dans laquelle l'huile ou les huiles de cultivar de menthe comprennent de la menthe « Banane ».

7. Composition selon la revendication 1, dans laquelle l'huile ou les huiles de cultivar de menthe comprennent de la menthe « Poire Sucrée ».

8. Composition selon la revendication 1, dans laquelle l'huile ou les huiles de cultivar de menthe comprennent de la menthe « Lavande ».

9. Composition selon la revendication 1, dans laquelle l'huile ou les huiles de cultivar de menthe comprennent de la menthe « Macho », de la menthe Julep, et des combinaisons de celles-ci, la composition comprenant en outre de la menthe poivrée.

10. Composition selon la revendication 1, dans laquelle l'huile ou les huiles de cultivar de menthe comprennent de la menthe « Gingembre », de la menthe « Lavande », et des combinaisons de celles-ci, la composition comprenant en outre de la menthe poivrée.

11. Composition selon l'une quelconque des revendications précédentes, qui comprend en outre un agent rafraîchissant choisi dans le groupe constitué par le menthol, le N-éthyl-p-menthane-3-carboxamide, le N,2,3-triméthyl-2-isopropylbutanamide, le 3-(p-menthane-3-carboxamido)acétate d'éthyle, le [(2-isopropyl-5-méthylcyclohexanecarbonyl)amino]acétate d'isopropyle, le glutarate de menthyle, le succinate de menthyle, le lactate de menthyle, le 3-1-menthoxypropane-1,2-diol, l'eucalyptol, et l'isopulégol, WS-5, WS-23 et des combinaisons de ceux-ci et la composition comprenant de 0,01 % à 5,0 % d'agent rafraîchissant en poids de la composition.

12. Composition selon la revendication 11, la composition comprenant de 0,02 % à 2,0 % d'agent rafraîchissant en poids de la composition.

13. Composition selon l'une quelconque des revendications précédentes, la composition étant formulée en un bien de consommation choisi dans le groupe constitué par un chewing-gum, un confiserie, une tablette, des pastilles, un dentifrice, une pâte dentifrice, un bain de bouche, un spray buccal, un produit de tabac, des substances pharmaceutiques, et des films comestibles.

14. Composition selon l'une quelconque des revendications précédentes, dans laquelle l'huile ou les huiles de cultivar de menthe sont encapsulées.

15. Composition selon l'une quelconque des revendications précédentes, comprenant en outre un arôme de fruit.

16. Composition selon l'une quelconque des revendications précédentes, comprenant en outre un édulcorant.

17. Composition selon l'une quelconque des revendications précédentes, dans laquelle l'huile ou les huiles de cultivar de menthe sont présentes en une quantité efficace pour apporter une note sensorielle secondaire choisie dans le groupe constitué par une note de vanille, poivrée, crayeuse, verte, de feuille, de pin, d'huile de poisson, d'agrume, de citron, crémeuse, boisée, de plantes, de peau blanche de citron, de plastique, métallique, de menthe au caramel, épicée, de miel, de thé vert, de zeste de citron et des combinaisons de celles-ci.
